# EUROPEAN PATENT SPECIFICATION

(11) **EP 0 989 112 B1**
(45) Date of publication and mention of the grant of the patent: **14.04.2004**
(21) Application number: 99124756.0
(22) Date of filing: 30.08.1995
(51) Int. Cl.: C07C 69/94

(54) **Intermediates for the preparation of guanidinomethyl cyclohexane carboxylic acid ester derivatives**
Zwischenprodukte für die Herstellung von Guanidinomethylcyclohexancarbonsäure-Estern
Intermèdiaires pour la préparation d'esters de l'acide guanidinométhylcyclohexane

(30) Priority: 30.08.1994 JP 24348994; 30.08.1994 JP 24349094; 05.09.1994 JP 24827094; 09.09.1994 JP 25265594
(43) Date of publication of application: 29.03.2000
(62) Divisional of application: 95930012.0
(73) Proprietor: Nagase ChemteX Corporation, Osaka 550-8668 (JP)
(72) Inventor: Komoda, Osamu, Itami-shi, Hyogo 664 (JP); Fujiwara, Hiromichi, Itami-shi, Hyogo 664 (JP); Yanagi, Toshiharu, Itami-shi, Hyogo 664 (JP)
(74) Representative: Vossius, Corinna

(56) References cited:
- JP-A- 57 016 856
- US-A- 2 092 724
- US-A- 2 092 725
- US-A- 2 584 160
- US-A- 4 798 680
- US-A- 4 844 835
- BRIAN A JONES ET AL: "Synthesis of smectic liquid-crystalline polysiloxanes from biphenylcarboxylate esters and their use as stationary phases for high-resolution gas chromatography" JOURNAL OF ORGANIC CHEMISTRY., vol. 49, no. 25, 1984, pages 4947-51, XP002147055 AMERICAN CHEMICAL SOCIETY. EASTON., US ISSN: 0022-3263
- TOMOYA KITAZUME ET AL: "Design and synthesis of new fluorinated ferroelectric liquid crystalline polymers" JOURNAL OF THE AMERICAN CHEMICAL SOCIETY., vol. 112, no. 18, 1990, pages 6608-15, XP002147056 AMERICAN CHEMICAL SOCIETY, WASHINGTON, DC., US ISSN: 0002-7863
- CHEMICAL ABSTRACTS, vol. 51, no. 4, 25 February 1957 (1957-02-25) Columbus, Ohio, US; abstract no. 2793g, SHOTARO NAKAJIMA ET AL: "Preparation of 3-phenylsalicylamide derivatives" page 1957; column 1; XP002147058 & J. PHARM. SOC. JAPAN, vol. 76, - 1956 pages 1211-13,
- KOSAKU HIROTA, YUKIO KITADE, SHIGEO SENDA: "Pyrimidine derivatives and related compounds. 39. A novel cycloaromatization reaction of 5-formyl-1,3-dimethyluracil with three-carbon nucleophiles. Synthesis of substituted 4-hydroxybenzoates" JOURNAL OF ORGANIC CHEMISTRY., vol. 46, no. 20, 1981, pages 3949-53, XP002147057 AMERICAN CHEMICAL SOCIETY. EASTON., US ISSN: 0022-3263
- Beilstein Registry No. 2502439
- Beilstein Registry No. 6775904
- Beilstein Registry No. 2093239

## Description

### 1. Field of the Invention

The present invention is related to novel and valuable intermediate compounds which can be used for the preparation of novel compounds comprising an antibacterial action, especially with a strong antibacterial action against Helicobacter pylori, and pharmaceutically acceptable salts thereof.

### 2. Background

Helicobacter pylori has been a remarked bacterium for investigating lesions of the stomach and duodenum for the report by Wallen and Marshal, in Australia, in 1983 (Lancet ii: 1437-1442 (1983)). It has been known that Helicobacter pylori is a helicoid microaerophile gram-negative bacillus, adjusts its life environment by producing urease, and lives in the tunica mucosa of the stomach and duodenum, and also causes or recauses inflammations or ulcerations.

It was reported that the United States Public Health Research Institute presented the recommendation to use a combination of antiulcerative agents and antibacterial agents for extermination of Helicobacter pylori (Chem. Indust. Daily Rep. Heisei 6.7.18) because Helicobacter pylori is considered to be strong by correlated with crisis and recurrence of peptic ulcer.

Antibiotics such as amoxicillin, cephalexin, clarislomycin (Jap. Clinic, vol. 51, No. 12, 1993), and synthetic antibacterial agents such as, ophroxacine, cyprofloxane, (APMIS, Suppl.,1007-1014, 1992) have been known as usable compounds for extermination of Helicobacter pylori and bacteria expulsion. Minocyline has also been known to be used for diseases caused by Helicobacter pylori, such as gastritis (Japanese Patent Publication No. Hei 6-508635 (95/508635)).

In Japan too, while the relation between Helicobacter pylori and peptic ulcer has been studied from now on, a movement has been raised, which investigates the response of Helicobacter pylori to the combination of antibiotics such as amoxicillin and omeplazol, lansoplazol which inhibit the activity of a proton pump and are used in clinics as antiulceration agents.

Since Helicobacter pylori is a bacterium producing urease and adjusts its life environment with the urease activity, a method for solving the above problem by using compounds with an inhibitory action against the above urease activity has been known. For example, Japanese Patent Laid-Open No. 4-217950/1992 reports that the aminomethylcyclohexane carboxylic acid phenyl ester group comprises are inhibitory activity against the urease activity, and also an inhibitory action of protease and, therefore it discloses compounds useful as drugs which are antiulceration agents. In the above publication, a compound of which an amino group was changed to a guanidino group, or a compound shown in the following Formula (XIV) was disclosed, and it was reported that the above mentioned compounds showed 88.7% of ulceration restraining ratio against an ethanol hydrochloride ulceration.

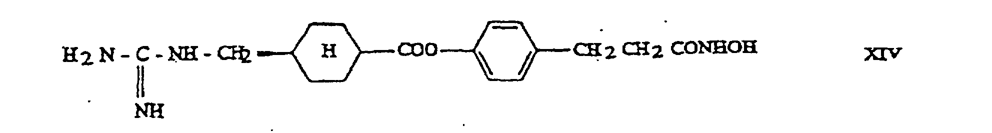

However, the above publication does not clarify the urease inhibition activity of the compounds.

Japanese Patent Publication No.57-16 856/1982 discloses guanidino methyl cyclohexane-carboxylic acid (GMCHA) esters made by reacting GMCHA with an R-substituted phenol, wherein said phenol may be biphenol when R is phenyl.

Further, in Japanese Patent Laid-Open No. 7-118153/1995, 2-[4[(3-methoxypropoxy)-3-methylpyridine-2-yl]methylsulfinyl-1H-benzimidazol, shown in Formula (XV) was disclosed as a compound with the inhibitory activity against urease.

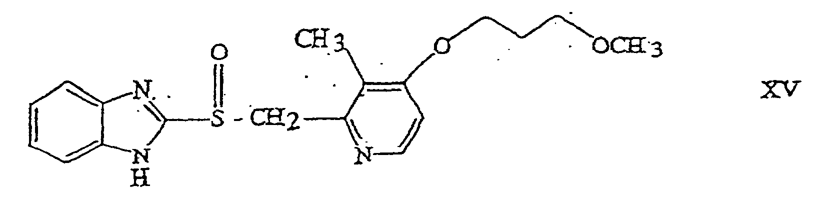

As administered antibiotics and synthetic antibacterial agents pass through the digestive tract, are absorbed in intestine, are metabolically-distributed among going into blood, are excreted with feces and so on, the above drugs when passing through the intestine cause extinction of many bacteria living in the intestine and disturb the balance of enterobacteria. Therefore, the longer-term dosage of such drugs should be avoided. Accordingly, compounds which comprise selectively strong antibacterial agents against Helicobacter pylori have been desired.

As Helicobacter pylori is a bacterium living in the stomach, the antibacterial activity of compounds having an antibacterial activity component which acts effectively in the stomach, becomes weak as the compounds move from the duodenum to the small intestine and, after all, it would be expected that the antibacterial activity vanishes. A compound called benexarte hydrochloride has been known as a compound which is chemically stable in the stomach, and is decomposed as moving to the small intestine (Progress in Medicine vol. 6, extra edition No. 1, 442 (1986)). It is is reported that the anti-Helicobacter pylori activity of this compound was MIC 25-50 µg/ml (Disease of systema digestorium and Helicobacter pylori, P 91, Medical Review).

The present inventors took into consideration the above mentioned background and studied compounds with specific and effective antibacterial activity against Helicobacter pylori and have achieved the present invention. The present invention provides novel and valuable intermediate compounds which can be used for the preparation of novel compounds comprising superior growth inhibition ability against Helicobacter pylori, but do not have an activity against Esylhiacori, Staphylococcus aureus, methacycline resistant bacteria, and so on, and are extremely speedy decomposed by actions of decomposition enzymes in the intestinum or blood.

U.S. Patent No. 4,220,662 discloses guanidino methyl cyclohexane carboxylic acid. As its ester, U.S. Patent No. 4,348,410 discloses a phenylester substituted with a group selected from halogen, lower alkoxy, formyl, lower alkanoylphenyl or a group of the formula -(CH2)nCOOR3 which was useful as an anti-ulceration agent and as a protease inhibitor, wherein R3 of said formula -(CH2)nCOOR3 is hydrogen, lower alkyl, phenyl, anisyle or alkoxycarbonylmethyl, and n is from 0 to 2. U.S. Patent No. 4,478,995 discloses that 6-benzyloxy carbonylphenyl ester was a useful antiulceration agent. Furthermore, it has also been well-known that such esters are useful as proteolytic enzyme inhibitors or antiulceration agents (Japanese Patent Publication No. 63-24988/1988, Japanese Patent Publication No. 63-24994/1988, Japanese Patent Publication No. 63-1940/1988, Japanese Patent Publication No. 63-32065/1988, Japanese Patent Publication No. 63-2255/1988, Japanese Patent Laid-Open No. 57-48960/1982, Japanese Patent Publication No. 64-2102/1989, Japanese Patent Publication No. 63-46743/1988, Japanese Patent Publication No. 64-2103/1989, Japanese Patent Publication No. 2-4588/1990, Japanese Patent Publication No. 64-2089/1989, Japanese Patent Publication No. 64-2086/1989, Japanese Patent Publication No. 64-2084/1989).

Moreover, it has also been well-known that a phenyl ester which is substituted with a halogen, lower alkyl, cyano, phenyl, benzyloxycarbonyl or phenoxycarbonyl group is useful against Escherichia coli (M. Kato et al., Biol. Pharma. Bull., 16 (2), 120-124 (1993)).

### 3. Detailed description of the Invention

The present invention relates to compounds of the general Formula (VIII) 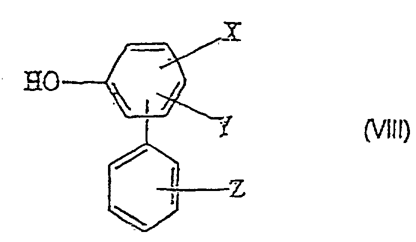 wherein X is hydrogen or halogen,
Y is hydrogen or substituted or unsubstituted aralkyloxycarbonyl group having 8 - 19 carbon atoms, wherein the aryl group may be substituted by one or more groups selected from alkyl groups having 1 to 10 carbon atoms, alkoxy having 1 to 10 carbon atoms, trihalogenomethyl or halogen,
Z is hydrogen, substituted or unsubstituted aralkyloxycarbonyl group having 8 - 19 carbon atoms, wherein the aryl group may be substituted by one or more groups selected from alkyl groups having 1 to 10 carbon atoms, alkoxy having 1 to 10 carbon atoms, trihalogenomethyl or halogen, or
Z is alkoxycarbonyl group having 2 - 19 carbon atoms, substituted in the alkyl group by a cycloalkyl group having 3 to 18 carbon atoms wherein at least one of Y or Z is not hydrogen.

Furthermore, the present invention relates to the compound 4-(4-hydroxyphenyl) benzoate [3-(4-fluorophenyloxy)propylester].

The compounds of the present invention are novel and valuable intermediate compounds which can be used for the preparation of antibacterial agents against Helicobacter pylori comprising guanidinomethyl cyclohexane carboxylic acid biphenyl ester which are shown in the following general Formula (II) and pharmaceutically acceptable salts thereof. 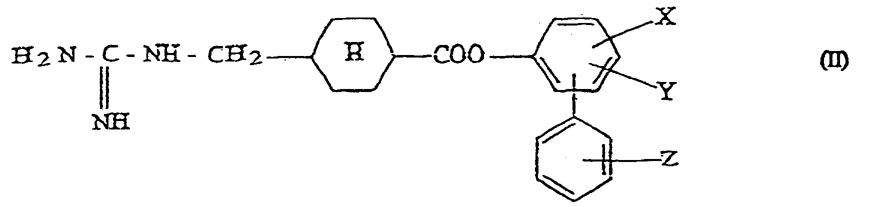 wherein X, Y and Z are defined as in Formula (VIII).

Halogen according to the present invention includes chlorine, iodine, bromine and fluorine.

A preferred alkyl group having one to ten carbon atoms is an alkyl group having from one to five carbon atoms, and may be linear or branched. Examples include methyl group, ethyl group, n-propyl group, i-propyl group, n-butyl group, i-butyl group, t-butyl group and so on.

A preferred alkoxy group having one to ten carbon atoms is an alkoxy group having one to five carbon atoms and may be linear or branched. Examples include methoxy group, ethoxy group, n-propoxy group and so on.

A preferred cycloalkyl group having three to eighteen carbon atoms is a cycloalkyl group having three to ten carbon atoms. Examples include cyclopropyl group, cyclobutyl group, cyclohexyl group and so on.

A preferred substituted alkoxy carbonyl group having two to nineteen carbon atoms is a substituted alkoxy carbonyl group having two to eleven carbon atoms and may be linear or branched. The alkoxycarbonyl group is substituted with a cycloalkyl group having three to eighteen carbon atoms, and preferably five to ten carbon atoms. Exemplary alkoxycarbonyl groups include cyclopropylmethyloxycarbonyl, cyclobutylmethyloxycarbonyl, cyclohexylethyloxycarbonyl, cyclohexylmethyloxycarbonyl, cyclohexylpropyloxycarbonyl, n-butylcyclohexylmethyloxycarbonyl.

A preferred substitued or unsubstituted aralkyloxycarbonyl group having eight to nineteen carbon atoms is a substituted or unsubstituted aralkyloxycarbonyl group having eight to thirteen carbon atoms, and has a linear or branched alkyl group. Suitable substituents of substituted aralkyloxycarbonyl groups are, e.g., halogen, alkyl group having one to ten carbon atoms, preferably one to five carbon atoms, alkoxy group having one to ten carbon atoms, preferably one to five carbon atoms, trihalogenomethyl. Exemplary substituted or unsubstituted aralkyloxycarbonyl groups include benzyloxycarbonyl, fluorobenzyloxycarbonyl, chlorobenzyloxycarbonyl, ethylbenzyloxycarbonyl, methylbenzyloxycarbonyl, propylbenzyloxycarbonyl, t-butylbenzyloxycarbonyl, methoxybenzyloxycarbonyl, trifluoromethylbenzyloxycarbonyl, dimethylbenzyloxycarbonyl, chlorophenylethyloxycarbonyl, methylphenylethyloxycarbonyl, phenylethyloxycarbonyl, phenylpropyloxycarbonyl.

Guanidinomethyl cyclohexane carboxylic acid ester of Formula (II) or the pharmaceutically acceptable salts thereof can be produced by the reaction between guanidinomethyl cyclohexane carboxylic acid shown as Formula (V) or the reactive derivatives thereof and the compounds of Formula (VIII) in a suitable solvent.

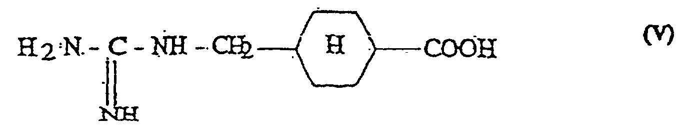

The suitable solvent for the above reaction can be any solvent which does not react with the starting materials. As such solvents, tetrahydrofuran, dioxane, isopropyl ether, ethylene glycol dimethyl ether, benzene, toluene, xylene, hexane, heptane, octane, petroleum ether, dichloroethane, acetonitrile, dimethylformamide, pyridine, triethylamine, dimethylaniline or mixture thereof and so on may be used.

As reactive derivative of the compound of Formula (V), for example, acid halide (chloride, bromide etc.), active ester (with p-nitrophenol etc.), acid anhydride, mixed acid anhydride (with ethyl chlorocarbonate, acetyl chloride, pivalic acid, POCl₃ etc.), and the reaction product with 1,1'-sulfinidimidazole, 1,1'-carbodiimidazole and so on may be used.

Condensation agents may be used to make react the compound of Formula (V) having the free carboxyl group with the compound of Formula (VIII). As suitable condensation agents, for example, dicyclohexylcarbodiimide, sulfuric acid, hydrogen chloride, toluensulfonic acid, thionyl chloride, phosphorus chloride, boron trifluoride and so on may be used.

Further, a reactive derivative of the compound of Formula (VIII) may be used to react with the compound of Formula (V). Exemplary reactive derivatives of the compound of Formula (VIII) are, e.g., chloro sulfate derivatives derived from thionylchloride, or sulfurous esters shown as Formula (VII).

**Ar - O - SO - O - Ar** (VII)

In the above Formulas (II) and (VIII), positions of the benzene ring having substituent Z can be any position of ortho, meta and para.

The antibacterial action of the compounds of Formula (II) prepared by using the compounds of Formula (VIII) of the present invention are examined as follows:

1. Minimum Inhibitory Concentration (MIC) was measured in accordance with the standard method of Japanese Chemotherapy Academy (Chemotherapy, 29: 76-79, 1981).

### 1) Measuring Methods of Sensitivity

Sensitivity was measured with an agar plating dilution using Brain-Heart Infusion agar (Difco Co.) which was added 0.1% of cyclodextrin.

### 2) Concentrations of Antibacterial Agents

The prepared concentrations were 25, 12.5, 6.25, 3.13, 1.56, 0.78, 0.39, 0.20, 0.10, 0.05, 0.025 and 0.0125 µg/ml.

10,000 µg/ml solutions of the compounds which were obtained in the examples described below were prepared by dissolving in dimethylsulfoxide. The solutions were diluted with sterilized distilled water to 250,125, 62.5, 31.3,15.6, 7.8, 3.9, 2.0,1.0,0.5,0.25 and 0.125 µg/ml. Each of 1 ml of these solutions was dispensed in share, after sterilization, 9 ml of sensitivity measuring medium which was kept at 50°C was added to each, and they were sufficiently mixed. The mixture was used as a plate for measuring sensitivity.

### 3) Bacterial Solution for Inoculation

Brain Heart Infusion agar medium (Difco Co.), to which 0.1% of cyclodextrin was added, was used as bacteria growth medium. The bacteria were cultured under microair at 37°C for three days, and the number of bacteria adjusted to about 106/ml. This was used as a bacterial solution for inoculation.

### 4) Method of Inoculating Bacteria

The medium was spread in each square about 2cm with a nichromic line loop.

### 5) Period and Temperature of the Culture

The medium was cultured under microair for three days at 37°C.

### 6) Evaluation

The minimum concentration which completely inhibited growth of the bacteria in the medium was judged as the value of MIC.

### 2. Minimum Inhibitory Concentration (MIC) and Minimum Bacteria sterilizeory

Concentration (MBC) against Helicobacter pylori were measured in accordance with the liquid medium dilution.

1,000 µg/ml solutions of the compounds which were obtained in the examples described below were prepared by dissolving in dimethylsulfoxide (DMSO). Distilled water was added to the solutions to prepare a double dilution system (200, 100, ..., 0.05 µg/ml). 1 ml of Brain-Heart Infusion broth (Difco Co.) to which 0.1 % of cyclodextrin was added and then was prepared at double concentrations, was added to each of 1 ml of the above DMSO solutions and mixed. 10 µl of Helicobacter pylori bacterial solution, which was cultured at 37°C for three days and then diluted and prepared with the above mentioned liquid medium, was added to the mixtures to adjust the volume to 10⁵ CFU/ml. The mixture was cultured in a multi-gas incubator (10% CO₂, 5% O₂, 85% N₂) at 37°C for three days and the minimum concentration of the compounds at which no growth of the bacteria was recognized with naked eyes were the values of MIC.

After the evaluation of MIC, each 100 µl of the compounds in each test tube were added to 4 ml of new liquid medium respectively. After culturing by the same method as described above, the minimum concentration of the compounds at which no growth with the naked eyes was recognized were the values of MBC.

The present invention will be described more specifically with several examples. The following examples do not limit the present invention.

### 3. Best modes of operation of the Invention

As comparative samples, the minimum inhibitory concentration (MIC) of the following well-known compounds other than the compounds of the examples were measured by the same procedure as above mentioned.
Compound 1: trans-4-guanidinomethyl cyclohexane carboxylic acid (4-phenylphenylester) hydrochloride
Compound 2: trans-4-guanidinomethyl cyclohexane carboxylic acid (2-phenylphenylester) hydrochloride

The values of MIC of the compounds obtained in Examples 1-15 and Compounds 1 and 2 are shown in Table 1, and the values of MIC of the compounds of Examples 16-23 are shown in Table 4.

As a comparison, MICs of ophroxacine and amoxicillin were measured and the results are shown in Table 1, 4, and 5. The antibacterial activity against Escherichia coli of the compounds of Examples 1-38 and Compounds 1-2, was also measured in accordance with the standard method of Japanese Chemotherapy Academy. The results are shown in corresponding tables.

The antibacterial activity of Compound 1 was measured using medium with 7% horse's defiber blood, which is the product been on the market, instead of 0.1% cyclodextrin used as the sensivity of measuring medium for the measurement of antibacterial activity. As the results, the values of MIC were >25 µg/ml and >25 µg/ml respectively.

For reference, the value of MIC of benexart hydrochloride was >100 µg/ml.

Moreover, each value of MBC of the compounds of Examples 1, 5-15 and Compound 1 against clinical segregation strains was measured in accordance with the above mentioned method. Each result is shown in Table 2.

Further more, MIC and MBC of the compounds of Examples 5 to 7 were measured using the liquid medium, and the results are shown in Table 3.

MIC of the compounds of Examples 1, 5, 8, 11, 18 and 22 was measured. The results are shown in Table 5.

### Example 1

Cooling down with ice, 1.11 g of thionyl chloride was added by dropping to a mixture of 4-(4-hydroxyphenyl)benzyl benzoate 5.68 g, pyridine 1.48 g and dried tetrahydrofuran 50 ml. After the mixture was stirred for 4 hours at room temperature, precipitated crystals were collected by filtration. The filtrate was concentrated under reduced pressure and an oily product was obtained. 30 ml of dried pyridine was added to the oily product and the mixed oily product was cooled down with ice. 2.00 g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration and they were washed with acetone. 3.95 g of trans-4-guanidinomethyl cyclohexane carboxylic acid [4-(4-benzyloxycarbonylphenyl) phenylester] · hydrochloride was obtained.
mp 198-201°C; NMR (DMSO-d₆) δ 0.8-3.3 (12H,m), 5.41 (2H,s), 7.0-8.3 (18H,m)

The value of MIC against bacillus subtilis ATCC 6633 measured in accordance with the standard method of Japanese Chemotherapy Academy was >25 µg/ml.

### Example 2

Cooling down with ice, 1.44 g of thionyl chloride was added by dropping to a mixture of 3-phenylsalicylate benzylester 7.00 g, dried pyridine 1.92 g and dried tetrahydrofuran 30 ml. After the mixture was stirred for 3 hours at room temperature, precipitated crystals were collected by filtration. The filtrate was concentrated under reduced pressure and oily product was obtained. 30 ml of dried pyridine was added to the oily product and the mixed oily product was cooled down with ice. 2.46 g of trans-4-guanidinomethyl cyclohexane carboxylic acid · hydrochloride was added to the cooled mixture. After stirring this mixture during night cooling down with ice, the mixture was concentrated under reduced pressure, acetone was added, and stirred. The supernatant was removed by decantation and acetone was added again. The solution was stirred during night, and precipitated crystals were collected by filtration, and washed with acetone. 2.13g of trans-4-guanidinomethyl cyclohexane carboxylic acid (2-benzyloxycarbonyl-6-phenylphenylester) · hydrochloride was obtained.
mp 81-85°C; NMR (DMSO-d₆) δ 0.6-3.2 (12H,m), 5.28 (2H,s), 6.7-8.2 (18H,m)

### Example 3

Cooling down with ice, 0.56 g of thionyl chloride was added by dropping to a mixture of 3-phenylsalicylate (4-fluorobenzylester) 3.01g, pyridine 0.74 g and dried tetrahydrofuran 20 ml. After the mixture was stirred for 3 hours at room temperature, precipitated crystals were collected by filtration. The filtrate was concentrated under reduced pressure and oily product was obtained. 20 ml of dried pyridine was added to the oily product and the mixed oily product was cooled down with ice. 1.00 g of trans-4-guanidinomethyl cyclohexane carboxylic acid hydrochloride was added to the cooled mixture. After stirring this mixture during night cooling down with ice, the mixed oily product was concentrated under reduced pressure. After isopropyl ether was added to the residue and stirred, the supernatant was removed by decantation. 80ml of water was added to the solution, and 0.45g of potassium hydrogencarbonate was added cooling down with ice, and stirred during night cooling down with ice. After 80ml of ethanol was added to the solution cooling down with ice, precipitated crystals were collected by filtration, and washed in order with water, ethanol and acetone. 0.45g of carbonate was obtained, and the carbonate was added into methanol. 0.08g of methanesulfonic acid was added to the mixture cooling down with ice and concentrated under reduced pressure. After acetone was added to the residue and stirred, precipitated crystals were collected by filtration. 0.42g of trans-4-guanidinomethyl cyclohexane carboxylic acid [2-(4-fluorobenzyloxycarbonyl)-6-phenylphenyl ester] · methanesulfonate was obtained.
mp 198-200°C; NMR (DMSO-d₆) δ 0.5-3.2 (12H,m), 2.48 (3H,s), 5.28 (2H,s), 6.6-7.8 (15H,m), 7.92 (1H,d), 8.04 (1H,d)

### Example 4

Cooling down with ice, a mixture of trans-4- guanidinomethyl cyclohexane carboxylic acid · hydrochloride 1.80g, 5-bromo-3-phenylsalicylate (4-fluorobenzylester) 3.37g and pyridine 30ml was stirred. After 1.73g of N,N'-dicyclohexylcarbodiimido was added to the mixture, the mixture was stirred during night at room temperature. Precipitated crystals were collected by filtration, and the filtrate was concentrated under reduced pressure. 30ml of water was added to the residue, and 0.80g of potassium hydrogen carbonate was added cooling down with ice. After 80ml of ethanol was added to the solution cooling down with ice, precipitated crystals were collected by filtration, and washed in order with water, ethanol and acetone. 3.79g of carbonate was obtained. 0.69g of the carbonate was added into methanol, and 0.12g of methanesulfonic acid was added to the mixture cooled down with ice. Some insoluble substances were filtered, and the filtrate was concentrated under reduced pressure. After acetone was added to the residue and stirred, precipitated crystals were collected by filtration. They were washed with acetone and 0.36g of crude crystals were collected. The crude crystals were dispersed by isopropanol, collected by filtration, and washed with isopropanol. 0.22g of trans-4-guanidinomethyl cyclohexane carboxylic acid [4-bromo-2-(4-fluorobenzyloxycarbonyl)-6-phenylphenylester] · methanesulfonate was obtained.
mp 206-208°C; NMR (DMSO-d₆) δ 0.5-3.2 (12H,m), 2.48 (3H,s), 5.31 (2H,s), 6.5-7.7 (14H,m), 7.81 (1H,d), 8.09 (1H,d)

### Example 5

Cooling down with ice, 1.11g of thionyl chloride was added by dropping to a mixture of 4-(4-hydroxyphenyl)benzoate(4-methylbenzylester) 5.95g, pyridine 1.48g and dried tetrahydrofuran 30ml. After the mixture was stirred for 3 hours at room temperature, precipitated crystals were collected by filtration. The filtrate was concentrated under reduced pressure. 30 ml of dried pyridine was added to the residue and the mixed residue was cooled down with ice. 2.00g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration, and washed with acetone. The crystals were dispersed into isopropanol, collected by filtration, and washed with isopropanol. 3.19g of trans-4-guanidinomethyl cyclohexane carboxylic acid [4-[4-(4-methylbenzyloxy carbonyl) phenyl] phenylester] · hydrochloride was obtained.
mp 171-173°C; NMR (DMSO-d₆) δ 0.6-3.3 (12H,m), 2.31 (3H,s), 5.30 (2H,s), 6.7-8.4 (17H,m)

The value of MIC against bacillus subtilis ATCC 6633 measured in accordance with the standard method of Japanese Chemotherapy Academy was >25 µg/ml.

### Example 6

Cooling down with ice, 1.11g of thionyl chloride was added by dropping to a mixture of 4-(4-hydroxyphenyl)benzoate(4-chlorobenzylester) 6.35g, pyridine 1.48g and dried tetrahydrofuran 30ml. After the mixture was stirred for 3 hours at room temperature, precipitated crystals were collected by filtration. The filtrate was concentrated under reduced pressure. 30ml of dried pyridine was added to the residue and the mixture was cooled down with ice. 2.00g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration, and washed with acetone. The crude crystals were dispersed into isopropanol, collected by filtration, and washed with isopropanol. 1.08g of trans-4-guanidinomethyl cyclohexane carboxylic acid [4-[4-(4-chlorobenzyloxy carbonyl) phenyl] phenylester] · hydrochloride was obtained.
mp 179-182°C; NMR (DMSO-d₆) δ 0.6-3.3 (12H,m), 5.34 (2H,s), 6.7-8.5 (17H,m)

The value of MIC against bacillus subtilis ATCC 6633 measured in accordance with the standard method of Japanese Chemotherapy Academy was >25 µg/ml.

### Example 7

Cooling down with ice, 1.11g of thionyl chloride was added by dropping to a mixture of 4-(4-hydroxyphenyl)benzoate(4-fluorobenzylester) 6.02g, pyridine 1.48g and dried tetrahydrofuran 50ml. After the mixture was stirred for 4 hours at room temperature, precipitated crystals were collected by filtration. The filtrate was concentrated under reduced pressure and an oily product was obtained. 30ml of dried pyridine was added to the oily product and the mixed oily product was cooled down with ice. 2.00g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration, and washed with acetone. The crystals were dispersed into isopropanol, collected by filtration, and washed with isopropanol. 3.22g of trans-4-guanidinomethyl cyclohexane carboxylic acid [4-[4-(4-fluorobenzyloxycarbonyl) phenyl] phenylester] · hydrochloride was obtained.
mp 178-180°C; NMR (DMSO-d₆) δ 0.7-3.3 (12H,m), 5.36 (2H,s), 6.8-8.6 (17H,m)

The value of MIC against bacillus subtilis ATCC 6633 measured in accordance with the standard method of Japanese Chemotherapy Academy was 25 µg/ml.

### Example 8

Cooling down with ice, 1.11g of thionyl chloride was added by dropping to a mixture of 4-(4-hydroxyphenyl)benzoate(4-t-butylbenzylester) 6.73g, pyridine 1.48g and dried tetrahydrofuran 50ml. After the mixture was stirred for 4 hours at room temperature, precipitated crystals were collected by filtration. The filtrate was concentrated under reduced pressure and an oily product was obtained. 30ml of dried pyridine was added to the oily product and the mixed oily product was cooled down with ice. 2.00g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, the precipitated crystals were collected by filtration, and washed with acetone. The crude crystals were dispersed into isopropanol, collected by filtration, and washed with isopropanol. 1.81 g of trans-4-guanidinomethyl cyclohexane carboxylic acid [4-[4-(4-t-butylbenzyloxy carbonyl) phenyl] phenylester] · hydrochloride was obtained.
mp 186-187°C; NMR (DMSO-d₆) δ 0.7-3.3 (21H,m), 5.36 (2H,s), 6.8-8.5 (17H,m)

The value of MIC against bacillus subtilis ATCC 6633 measured in accordance with the standard method of Japanese Chemotherapy Academy was >25 µg/ml.

### Example 9

Cooling down with ice, 1.11g of thionyl chloride was added by dropping to a mixture of 4-(4-hydroxyphenyl)benzoate(4-methoxybenzylester) 6.25g, pyridine 1.48g and dried tetrahydrofuran 50ml. After the mixture was stirred for 4 hours at room temperature, precipitated crystals were collected by filtration. The filtrate was concentrated under reduced pressure and an oily product was obtained. 30ml of dried pyridine was added to the oily product and the mixed oily product was cooled down with ice. 2.00g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration, and washed with acetone. The crude crystals were dispersed into isopropanol, collected by filtration, and washed with isopropanol. 3.62g of trans-4-guanidinomethyl cyclohexane carboxylic acid [4-[4-(4-methoxybenzyloxy carbonyl) phenyl] phenylester] · hydrochloride was obtained.
mp 180-183°C; NMR (DMSO-d₆) δ 0.6-3.4 (12H,m), 3.75 (3H,s), 5.30 (2H,s), 6.7-8.5 (17H,m)

The value of MIC against bacillus subtilis ATCC 6633 measured in accordance with the standard method of Japanese Chemotherapy Academy was 25 µg/ml.

### Example 10

Cooling down with ice, 1.11 g of thionyl chloride was added by dropping to a mixture of 4-(4-hydroxyphenyl)benzoate(2-fluorobenzylester) 6.03g, pyridine 1.48g and dried tetrahydrofuran 50ml. After the mixture was stirred for 4 hours at room temperature, precipitated crystals were collected by filtration. The filtrate was concentrated under reduced pressure and an oily product was obtained. 30ml of dried pyridine was added to the oily product and the mixed oily product was cooled down with ice. 2.00g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration, and washed with acetone. The crude crystals were dispersed into isopropanol, collected by filtration, and washed with isopropanol. 2.93g of trans-4-guanidinomethyl cyclohexane carboxylic acid [4-[4-(2-fluorobenzyloxycarbonyl)phenyl] phenylester] · hydrochloride was obtained.
mp 180-183°C; NMR (DMSO-d₆) δ 0.6-3.3 (12H,m), 5.45 (2H,s), 6.8-8.5 (17H,m)

The value of MIC against bacillus subtilis ATCC 6633 measured in accordance with the standard method of Japanese Chemotherapy Academy was >25 µg/ml.

### Example 11

Cooling down with ice, 1.11g of thionyl chloride was added by dropping to a mixture of 4-(4-hydroxyphenyl)benzoate(4-trifluoromethyl benzylester) 6.95g, pyridine 1.48g and dried tetrahydrofuran 50ml. After the mixture was stirred for 4 hours at room temperature, precipitated crystals were collected by filtration. The filtrate was concentrated under reduced pressure and an oily product was obtained. 30ml of dried pyridine was added to the oily product and the mixed oily product was cooled down with ice. 2.00g of trans-4-guanidinomethyl cyclohexane carboxylic acid - hydrochloride was added to the cooled mixture. After stirring this mixture during night at room temperature, the mixture was concentrated under reduced pressure. After acetone was added to the residue, precipitated crystals were collected by filtration, and washed with acetone. The crude crystals were dispersed into isopropanol, collected by filtration, and washed with isopropanol. 3.20g of trans-4-guanidinomethyl cyclohexane carboxylic acid [4-[4-(4-trifluoromethyl benzyloxycarbonyl) phenyl] phenylester] · hydrochloride was obtained.
mp 188-190°C; NMR (DMSO-d₆) δ 0.6-3.4 (12H,m), 5.50 (2H,s), 6.9-8.5 (17H,m)

The value of MIC against bacillus subtilis ATCC 6633 measured in accordance with the standard method of Japanese Chemotherapy Academy was >25 µg/ml.

### Example 12

Cooling down with ice, 0.83g of thionyl chloride was added by dropping to a mixture of 4-(4-hydroxyphenyl)benzoate cyclohexylmethylester 4.35g, dried pyridine 1.11g and dried tetrahydrofuran 30ml. After the mixture was stirred for 3 hours at room temperature, precipitated crystals were collected by filtration. The filtrate was concentrated under reduced pressure. 30ml of dried pyridine was added to the residue and the mixture was cooled down with ice. 1.50g of trans-4-guanidinomethyl cyclohexane carboxylic acid · hydrochloride was added to the cooled mixture. After stirring this mixture during night cooling down with water, the mixture was concentrated under reduced pressure. After acetone was added to the residue, precipitated crystals were collected by filtration, and washed with acetone. 2.85 g of crude crystals were obtained. The crude crystals were dispersed into isopropanol, collected by filtration, and washed with isopropanol. 2.44g of trans-4-guanidinomethyl cyclohexane carboxylic acid [4-(4-cyclohexylmethyloxy carbonylphenyl) phenylester] · hydrochloride was obtained.
mp 192-196°C; NMR (DMSO-d₆) δ 0.5-3.3 (12H,m), 4.10 (2H,d), 6.9-8.5 (13H,m)

The value of MIC against bacillus subtilis ATCC 6633 measured in accordance with the standard method of Japanese Chemotherapy Academy was 25 µg/ml.

### Example 13

Cooling down with ice, 0.30g of thionyl chloride was added by dropping to a mixture of 4-(3-bromo-4-hydroxyphenyl)benzoate(4-methyl benzylester) 2.00g, dried pyridine 0.40g and dried tetrahydrofuran 10ml. After the mixture was stirred for 3 hours at room temperature, precipitated crystals were collected by filtration. The filtrate was concentrated under reduced pressure. 10ml of dried pyridine was added to the residue and the mixture was cooled down with ice. 0.54g of trans-4-guanidinomethyl cyclohexane carboxylic acid · hydrochloride was added to the cooled mixture. After stirring this mixture during night at room temperature, the mixture was concentrated under reduced pressure. After acetone was added to the residue, precipitated crystals were collected by filtration, and washed with acetone. 0.79 g of crude crystals were obtained. The crude crystals were dispersed into isopropanol, collected by filtration, and washed with isopropanol. 0.60g of trans-4-guanidinomethyl cyclohexane carboxylic acid [2-bromo-4-[4-(4-methylbenzyloxycarbonyl) phenyl] phenylester] · hydrochloride was obtained.
mp 135-138°C; NMR (DMSO-d₆) δ 0.6-3.3 (12H,m), 2.33 (3H,s), 5.33 (2H,s), 6.4-8.6 (16H,m)

The value of MIC against bacillus subtilis ATCC 6633 measured in accordance with the standard method of Japanese Chemotherapy Academy was >25 µg/ml.

### Example 14

Cooling down with ice, 0.56g of thionyl chloride was added by dropping to a mixture of 4-(4-hydroxyphenyl)benzoate(2,4-dimethyl benzylester) 3.10g, dried pyridine 0.74g and dried tetrahydrofuran 15ml. After the mixture was stirred for 3 hours at room temperature, precipitated crystals were collected by filtration. The filtrate was concentrated under reduced pressure. 15ml of dried pyridine was added to the residue and the mixture was cooled down with ice. 1.00g of trans-4-guanidinomethyl cyclohexane carboxylic acid · hydrochloride was added to the cooled mixture. After stirring this mixture during night cooling down with water, the mixture was concentrated under reduced pressure. After acetone was added to the residue, precipitated crystals were collected by filtration, and washed with acetone. The crude crystals were dispersed into isopropanol, collected by filtration, and washed with isopropanol. 1.48g of trans-4-guanidinomethyl cyclohexane carboxylic acid [4-[4-(2,4-dimethyl benzyloxycarbonyl) phenyl] phenylester] · hydrochloride was obtained.
mp 177-180°C; NMR (DMSO-d₆) δ 0.6-3.3 (18H,m), 5.31 (2H,s), 6.5-8.5 (16H,m)

The value of MIC against bacillus subtilis ATCC 6633 measured in accordance with the standard method of Japanese Chemotherapy Academy was >25 µg/ml.

### Example 15

Cooling down with ice, 0.19g of thionyl chloride was added by dropping to a mixture of 4-(4-hydroxyphenyl)benzoate(2-phenylethylester) 1.01g, dried pyridine 0.25g and dried tetrahydrofuran 10ml. After the mixture was stirred for 3 hours at room temperature, precipitated crystals were collected by filtration. The filtrate was concentrated under reduced pressure. 10ml of dried pyridine was added to the residue and the mixture was cooled down with ice. 0.34g of trans-4-guanidinomethyl cyclohexane carboxylic acid · hydrochloride was added to the cooled mixture. After stirring this mixture during night cooling down with water, precipitated crystals were collected by filtration, and washed with acetone. Crude crystals were obtained and were dispersed into acetone, collected by filtration, and washed with acetone. 0.33g of trans-4-guanidinomethyl cyclohexane carboxylic acid [4-[4-(2-phenylethyloxycarbonyl)phenyl]phenylester] · hydrochloride was obtained.
mp 192-194°C; NMR (DMSO-d₆) δ 0.7-3.3 (14H,m), 4.49 (2H,t), 6.7-8.4 (18H,m)

The value of MIC against bacillus subtilis ATCC 6633 measured in accordance with the standard method of Japanese Chemotherapy Academy was >25 µg/ml.

### Example 16

Cooling down with ice, 0.61g of thionyl chloride was added by dropping to a mixture of 4-(4-hydroxyphenyl)benzoate(3-phenylpropylester) 3.41g, pyridine 0.81 g and dried tetrahydrofuran 50ml. After the mixture was stirred for 3 hours at room temperature, precipitated crystals were collected by filtration. The filtrate was concentrated under reduced pressure and an oily product was obtained. 30ml of dried pyridine was added to the oily product and cooled down with ice. 1.10g of trans-4-guanidinomethyl cyclohexane carboxylic acid · hydrochloride was added to the cooled mixture. After stirring this mixture during night at room temperature, the mixture was concentrated under reduced pressure and acetone was added to the residue. Precipitated crystals were collected by filtration, and washed with acetone. The obtained crystals were suspended in 30ml of 2-propanol, collected by filtration, and washed in order with 2-propanol and acetone. 1.75g of trans-4-guanidinomethyl cyclohexane carboxylic acid [4-[4-(3-phenylpropyloxycarbonyl)phenyl] phenylester] · hydrochloride was obtained.
mp 180-185°C; NMR (DMSO-d₆) δ 0.8-3.3 (16H,m), 4.30 (2H,t), 6.8-9.0 (18H,m)

### Example 17

Cooling down with ice, 0.56g of thionyl chloride was added by dropping to a mixture of 4-(4-hydroxyphenyl)benzoate[3-(4-fluorophenyloxy) propylester] 3.27g, pyridine 0.74g and dried tetrahydrofuran 50ml. After the mixture was stirred for 3 hours at room temperature, precipitated crystals were collected by filtration. The filtrate was concentrated under reduced pressure and white colored crystals were obtained. 30ml of dried pyridine was added to the white colored crystals and the mixture was cooled down with ice. 1.00g of trans-4-guanidinomethyl cyclohexane carboxylic acid · hydrochloride was added to the cooled mixture. After stirring this mixture during night at room temperature, the mixture was concentrated under reduced pressure and acetone was added to the residue. Precipitated crystals were collected by filtration, and washed with acetone. The obtained crystals were suspended in 30ml of 2-propanol, collected by filtration, and washed in order with 2-propanol and acetone. 1.59g of trans-4-guanidinomethyl cyclohexane carboxylic acid [4-[4-[3-(4-fluoro phenyloxy)propyloxycarbonyl]phenyl]phenylester] · hydrochloride was obtained.
mp 175-183°C; NMR (DMSO-d₆) δ 0.8-3.3 (14H,m), 4.21 (2H,t), 4.47 (2H,t), 6.8-8.7 (17H,m)

### Example 18

Cooling down with ice, 0.56g of thionyl chloride was added by dropping to a mixture of 4-(4-hydroxyphenyl) benzoate (2-cyclohexyl ethylester) 3.03g, pyridine 0.74g and dried tetrahydrofuran 50ml. After the mixture was stirred for 3 hours at room temperature, precipitated crystals were collected by filtration. The filtrate was concentrated under reduced pressure and an oily product was obtained. 30ml of dried pyridine was added to the oily product and cooled down with ice. 1.00g of trans-4-guanidinomethyl cyclohexane carboxylic acid · hydrochloride was added to the cooled mixture. After stirring this mixture during night at room temperature, precipitated crystals were collected by filtration, and washed in order with pyridine and acetone. The obtained crystals were suspended in 30ml of 2-propanol, collected by filtration, and washed in order with 2-propanol and acetone. 1.77g of trans-4-guanidinomethyl cyclohexane carboxylic acid [4-[4-(2-cyclohexylethyl oxycarbonyl)phenyl] phenylester] · hydrochloride was obtained.
mp 192-205°C; NMR (DMSO-d₆) δ 0.7-3.3 (25H,m), 4.31 (2H,t), 6.9-9.0 (13H,m)

### Example 19

Cooling down with ice, 0.28g of thionyl chloride was added by dropping to a mixture of 4-(4-hydroxyphenyl) benzoate (trans-4-n-butyl cyclohexylmethylester) 1.71g, pyridine 0.37g and dried tetrahydrofuran 30ml. After the mixture was stirred for 3 hours at room temperature, precipitated crystals were collected by filtration. The filtrate was concentrated under reduced pressure and an oily product was obtained. 30ml of dried pyridine was added to the oily product and cooled down with ice. 0.50g of trans-4-guanidinomethyl cyclohexane carboxylic acid · hydrochloride was added to the cooled mixture. After stirring this mixture during night at room temperature, the mixture was concentrated under reduced pressure and acetone was added to the residue. Precipitated crystals were collected by filtration, and washed with acetone. The obtained crystals were suspended in 30ml of 2-propanol, collected by filtration, and washed in order with 2-propanol and acetone. 0.43g of trans-4-guanidinomethyl cyclohexane carboxylic acid [4-[4-(trans-4-n- butylcyclohexylmethyloxycarbonyl)phenyl] phenylester] - hydrochloride was obtained.
mp 191-196°C; NMR (DMSO-d₆) δ 0.4-3.3 (31H,m), 3.9-4.4 (2H,m), 6.9-8.7 (13H,m)

### Example 20

Cooling down with ice, 5.55g of thionyl chloride was added by dropping to a mixture of 4-(4-hydroxyphenyl) benzoate (2-methylbenzylester) 29.72g, pyridine 7.38g and dried tetrahydrofuran 150ml. After the mixture was stirred for 4 hours at room temperature, precipitated crystals were collected by filtration. The filtrate was concentrated under reduced pressure and an oily product was obtained. 80ml of dried pyridine was added to the oily product and cooled down with ice. 10.0g of trans-4-guanidinomethyl cyclohexane carboxylic acid · hydrochloride was added to the cooled mixture in portions. After stirring this mixture during night at room temperature, the mixture was concentrated under reduced pressure and acetone was added to the residue. Precipitated crystals were collected by filtration, and washed with acetone. 22.43g of trans-4-guanidinomethyl cyclohexane carboxylic acid [4-[4-(2-methylbenzyloxy carbonyl)phenyl] phenylester] · hydrochloride was obtained.
mp 191-194°C; NMR (DMSO-d₆) δ 0.8-3.3 (12H,m), 2.39 (3H,s), 5.32 (2H,s), 6.9-8.2 (17H,m)

### Example 21

Cooling down with ice, 2.22g of thionyl chloride was added by dropping to a mixture of 4-(4-hydroxyphenyl) benzoate (3-methylbenzylester) 11.89g, pyridine 2.95g and dried tetrahydrofuran 100ml. After the mixture was stirred for 4.5 hours at room temperature, precipitated crystals were collected by filtration. The filtrate was concentrated under reduced pressure and white colored crystals were obtained. 30ml of dried pyridine was added to the white colored crystals and the mixture was cooled down with ice. 4.00g of trans-4-guanidinomethyl cyclohexane carboxylic acid · hydrochloride was added to the cooled mixture. After stirring this mixture during night at room temperature, the mixture was concentrated under reduced pressure and acetone was added to the residue. Precipitated crystals were collected by filtration, and washed with acetone. 8.62g of trans-4-guarudinomethyl cyclohexane carboxylic acid [4-[4-(3-methylbenzyloxycarbonyl)phenyl]phenylester] · hydrochloride was obtained.
mp 178-180°C; NMR (DMSO-d₆) δ 0.8-3.3 (12H,m), 2.33 (3H,s), 5.30 (2H,s), 6.9-8.3 (17H,m)

### Example 22

Cooling down with ice, 1.18g of thionyl chloride was added by dropping to a mixture of 4-(4-hydroxyphenyl) benzoate [2-(4-chloro phenyl)ethylester] 7.00g, pyridine 1.57g and dried tetrahydrofuran 100ml. After the mixture was stirred for 3 hours at room temperature, precipitated crystals were collected by filtration. The filtrate was concentrated under reduced pressure and crystals were obtained. 40ml of dried pyridine and 30ml of N,N-dimethylformamido were added to the crystals and cooled down with ice. 2.13g of trans-4-guanidinomethyl cyclohexane carboxylic acid · hydrochloride was added to the cooled mixture. After stirring this mixture during night at room temperature, the mixture was concentrated under reduced pressure and acetone was added to the residue. Precipitated crystals were collected by filtration, and washed with acetone. The obtained crystals were suspended in 30ml of 2-propanol, collected by filtration, and washed in order with 2-propanol and acetone. 3.84g of trans-4-guanidinomethyl cyclohexane carboxylic acid [4-[4-[2-(4-chlorophenyl)ethyloxycarbonyl)phenyl] phenylester] · hydrochloride was obtained.
mp 195-202°C; NMR (DMSO-d₆) δ 0.8-3.3 (14H,m), 4.47 (2H,t), 6.8-8.2 (17H,m)

### Example 23

Cooling down with ice, 1.25g of thionyl chloride was added by dropping to a mixture of 4-(4-hydroxyphenyl) benzoate [2-(4-methyl phenyl)ethylester] 7.00g, pyridine 1.67g and dried tetrahydrofuran 100ml. After the mixture was stirred for 3 hours at room temperature, precipitated crystals were collected by filtration. The filtrate was concentrated under reduced pressure and crystals were obtained. 40ml of dried pyridine and 30ml of N,N-dimethylformamido were added to the crystals and cooled down with ice. 2.26g of trans-4-guanidinomethyl cyclohexane carboxylic acid · hydrochloride was added to the cooled mixture. After stirring this mixture during night at room temperature, the mixture was concentrated under reduced pressure and acetone was added to the residue. Precipitated crystals were collected by filtration, and washed with acetone. The obtained crystals were suspended in 30ml of 2-propanol, collected by filtration, and washed in order with 2-propanol and acetone. 2.96g of
trans-4-guanidinomethyl cyclohexane carboxylic acid
[4-[4-[2-(4-methylphenyl)ethyloxycarbonyl)phenyl] phenylester] · hydrochloride was obtained.
mp 182-190°C; NMR (DMSO-d₆) δ 0.8-3.3 (14H,m), 2.23 (3H,s), 4.43 (2H,t), 6.8-8.1 (17H,m)

The raw compounds of Examples, which were shown as Formula (VIII), were prepared as follows:

### Sample 1

At room temperature, 0.7ml of concentrated sulfuric acid was added to 5.00g of 4-(4-hydroxyphenyl)benzoic acid suspended in 50ml of ethanol. After the mixture was refluxed with heating for 7 hours, the mixture was concentrated under reduced pressure. Water and ethyl acetate were added to the residue, and then the organic layer was washed in order with water, saturated sodium hydrogen carbonate and saturated sodium chloride solution. It was dried with anhydrous magnesium sulfate, and concentrated under reduced pressure. Obtained crude crystals were purified by silica gel column chromatography with toluene-ethyl acetate eluent. Obtained crystals were dispersed into hexane, collected by filtration and washed with hexane. 4-(4-hydroxyphenyl) benzoate ethylester 5.04g was obtained which is not part of the present invention.
mp 156-157°C

### Sample 2

2.25g of methanol solution including 28% sodium methylate was added to 2.50g of 4-(4-hydroxyphenyl) benzoic acid suspended in 10ml of methanol. After the mixture was stirred for 5 minutes, it was concentrated under reduced pressure. 10ml of N,N-dimethylformamide and 2.43g of benzyl- bromoamide were added to the residue. The mixture was stirred during night at room temperature, poured into 30ml of ice water, and extracted with 50ml of toluene. The organic layer was washed with in order saturated sodium hydrogen and water, dried with anhydrous magnesium sulfate, and concentrated under reduced pressure. Obtained crude crystals were purified by silica gel column chromatography with toluene-ethyl acetate eluent, and hexane was added. Precipitated crystals were collected by filtration, and washed with hexane. 4-(4-hydroxyphenyl) benzoate benzylester 2.60g was obtained.
mp 131-133°C

In accordance with the same procedures as above described the compounds shown as Formula (VIII) in Examples 2, 3, 5-12, 14 and 15 were prepared. The prepared compounds and the physical properties thereof are as follows.
3-phenylsalicylic acid benzylester: mp 69-70°C
3-phenylsalicylic acid (4-fluorobenzylester): mp 85-86°C
4-(4-hydroxyphenyl) benzoate (4-methylbenzylester): mp 128-130°C
4-(4-hydroxyphenyl) benzoate (4-chlorobenzylester): mp 138-140°C
4-(4-hydroxyphenyl) benzoate (4-fluorobenzylester): mp 157-158°C
4-(4-hydroxyphenyl) benzoate (4-t-butylbenzylester): mp 125-126°C
4-(4-hydroxyphenyl) benzoate (4-methoxybenzylester): mp 156-159°C
4-(4-hydroxyphenyl) benzoate (2-fluorobenzylester): mp 154-155°C
4-(4-hydroxyphenyl) benzoate (4-trifluoromethylbenzylester): mp 141-142°C
4-(4-hydroxyphenyl) benzoate cyclohexylmethylester: mp 172-176°C
4-(4-hydroxyphenyl) benzoate (2,4-dimethylbenzylester): mp 139-145°C
4-(4-hydroxyphenyl) benzoate (2-phenylethylester): mp 152-155°C

The raw compounds of Examples 16-23 were prepared by the same procedures as above mentioned or by the procedures applied above. The prepared compounds and the physical properties thereof are as follows.
4-(4-hydroxyphenyl) benzoate (3-phenylpropylester): mp 98-99°C
4-(4-hydroxyphenyl) benzoate [3-(4-fluorophenyloxy)propylester]: mp 151-153°C
4-(4-hydroxyphenyl) benzoate (2-cyclohexylethylester): mp 133-134°C
4-(4-hydroxyphenyl) benzoate (trans-4-n-butylcyclohexylmethylester): mp 130-131°C
4-(4-hydroxyphenyl) benzoate (2-methylbenzylester): mp 148-150°C
4-(4-hydroxyphenyl) benzoate (3-methylbenzylester): mp 98.5-99.5°C
4-(4-hydroxyphenyl) benzoate [2-(4-chlorophenyl)ethylester]: mp 177-180°C
4-(4-hydroxyphenyl) benzoate [2-(4-methylphenyl)ethylester]: mp 180-182°C

### Sample 3

N-bromosuccinimide 1.98g was added to a mixture of 3-phenylsalicylic acid (4-fluorobenzylester) 3.26g and N,N-dimethylformamide 15ml. After the mixture was stirred during night at room temperature, the mixture was poured into ice water and extracted with toluene. The organic layer was washed with water, dried with anhydrous magnesium sulfate, and concentrated under reduced pressure. The residue was purified by silica gel column chromatography with toluene-ethyl acetate eluent, and hexane was added. Precipitated crystals were collected by filtration, and washed with hexane. 5-bromo-3-phenylsalicylate (4-fluorobenzylester) 3.64g was obtained.
mp 87-88°C

### Sample 4

Keeping at lower than 35°C of internal temperature with ice water, N-bromosuccinimide 3.66g was added to a mixture of 4-(4-hydroxyphenyl) benzoate 4.00g and N,N-dimethylformamide 50ml. After the mixture was stirred during night at room temperature, the mixture was poured into ice water and extracted with chloroform. The organic layer was washed with water, dried with anhydrous magnesium sulfate, and concentrated under reduced pressure. Hexane was added to the residue and precipitated crystals were collected by filtration. 4.88g of crude crystals of 4-(3-bromo-4-hydroxyphenyl) benzoate were obtained. 40ml of methanol was added to 4.00g of the crude crystals and 2.63g of methanol solution including 28% sodium methylate was added by dropping to the mixture cooling down with ice. After the mixture was stirred for 1 hour at room temperature, the mixture was concentrated under reduced pressure. 80ml of N,N-dimethyl formamide and also, cooling down with ice, 2.78g of 4-methylbenzylbromide were added to the residue. After the mixture was stirred for 3 days at room temperature, the mixture was poured into ice water and extracted with toluene. The organic layer was washed in order with saturated sodium hydrogen carbonate and water, dried with anhydrous magnesium sulfate, and concentrated under reduced pressure. 4.98g of oily product was obtained. The oily product was purified by silica gel column chromatography with toluene-ethyl acetate eluent, and hexane was added. Precipitated crystals were collected by filtration, and washed with hexane. 4-(3-bromo-4-hydroxyphenyl) benzoate (4-methylbenzylester) 2.35g was obtained.
mp 93-94°C

**Table 1**

| Compounds | M I C (µg/ml) | | |
|---|---|---|---|
| | Objected Strain 1 | Objected Strain 2 | Objected Strain 3 |
| Example 1 | 0.78 | 0.39 | >25 |
| Example 2 | 6.25 | 3.13 | >25 |
| Example 3 | 6.25 | 6.25 | >25 |
| Example 4 | 6.25 | 6.25 | >25 |
| Example 5 | 0.39 | 0.39 | >25 |
| Example 6 | 0.78 | 0.39 | >25 |
| Example 7 | 0.78 | 0.78 | >25 |
| Example 8 | 0.78 | 0.39 | >25 |
| Example 9 | 0.78 | 0.78 | >25 |
| Example 10 | 0.39 | 0.39 | >25 |
| Example 11 | 0.39 | 0.78 | >25 |
| Example 12 | 0.20 | . 0.39 | >25 |
| Example 13 | 0.39 | 0.78 | >25 |
| Example 14 | 0.39 | 0.78 | >25 |
| Example 15 | 0.39 | 0.78 | >25 |
| Compound 1 | 3.13 | 0.78 | >25 |
| Compound 2 | 25 | 25 | >25 |
| Ophroxacine | 0.78 | 0.78 | 0.10 |
| Amoxicillin | 0.05 | 0.025 | 6.25 |
| Objected Strain 1: Helicobacter pylori ATCC 43504 | | | |
| Objected Strain 2: Helicobacter pylori ATCC 43629 | | | |
| Qbjected Strain 3: Escherichia coli NIH JC-2 | | | |

**Table 2**

| Compound | M I C[µg/ml] | | | | | | |
|---|---|---|---|---|---|---|---|
| | Strain 4 | Strain 5 | Strain 6 | Strain 7 | Strain 8 | Strain 9 | Strain 10 |
| Componnd1 | 0.39 | 1.56 | 0.39 | 0.78 | | | |
| Example 1 | 0.20 | 0.39 | 0.10 | 0.39 | | | |
| Example 5 | 0.10 | 0.39 | 0.10 | 0.20 | 0.20 | 0.39 | 0.39 |
| Example 6 | 0.10 | 0.39 | 0.10 | 0.20 | | | |
| Example 7 | 0.20 | 0.78 | 0.20 | 0.39 | | | |
| Example 8 | 0.20 | 0.39 | 0.20 | 0.20 | | | |
| Example 9 | 0.20 | 0.78 | 0.20 | 0.39 | | | |
| Example 10 | 0.20 | 0.78 | 0.20 | 0.39 | | | |
| Example 11 | 0.20 | 0.39 | 0.10 | 0.39 | | | |
| Example 12 | 0.20 | 0.39 | 0.20 | 0.39 | | | |
| Example 13 | | 0.39 | 0.20 | 0.39 | | | |
| Example 14 | 0.39 | 0.39 | 0.20 | 0.39 | | | |
| Example 15 | 0.20 | 0.39 | 0.10 | 0.20 | | | |
| Strain = Objected Strain | | | | | | | |
| Strain 4: Helicobacter pylori No.1 | | | | | | | |
| Strain 5: Helicobacter pylori No.2 | | | | | | | |
| Strain 6: Helicobacter pylori No. 3 | | | | | | | |
| Strain 7: Helicobacter pylori No.4 | | | | | | | |
| Strain 8: Helicobacter pylori No.19 | | | | | | | |
| Strain 9: Helicobacter pylori No. 20 | | | | | | | |
| Strain 10: Helicobacter pylori No. 28 | | | | | | | |

**Table 3**

| Compound | Objected Strain 1 | | Objected Strain 2 | | Objected Strain 4 | | Objected Strain 5 | | Objected Strain 6 | | Objected Strain 7 | |
|---|---|---|---|---|---|---|---|---|---|---|---|---|
| | MIC | MBC | MIC | MBC | MIC | MBC | MIC | MBC | MIC | MBC | MIC | MBC |
| Example 5 | 0.20 | 0.20 | 0.39 | 1.56 | 0.20 | 0.20 | 0.39 | 1.56 | 0.10 | 0.10 | 0.39 | 0.39 |
| Example 6 | 0.20 | 0.39 | 0.39 | 1.56 | 0.20 | 0.78 | 0.39 | 1.56 | 0.05 | 0.10 | 0.39 | 0.39 |
| Example 7 | 0.20 | 0.39 | 0.39 | 1.56 | 0.20 | 0.78 | 0.78 | 3.13 | 0.05 | 0.10 | 0.10 | 0.39 |
| The unit of MIC and MBC measurements is µg/ml. | | | | | | | | | | | | |
| Objected Strain 1:Helicobacter pylori ATCC 43504 | | | | | | | | | | | | |
| Objected Strain 2: Helicobacter pylori ATCC 43629 | | | | | | | | | | | | |
| Objected Strain 4: Helicobacter pylori No. 1 | | | | | | | | | | | | |
| Objected Strain 5: Helicobacter pylori Ho. 2 | | | | | | | | | | | | |
| Objected Strain 6: Helicobacter pylori No. 3 | | | | | | | | | | | | |
| Objected Strain 7: Helicobacter pylori No. 4 | | | | | | | | | | | | |

**Table 4**

| Compound | M I C(µg/ml) | | | | | | | | | |
|---|---|---|---|---|---|---|---|---|---|---|
| | Strain 1 | Strain 2 | Strain 3 | Strain 4 | Strain 5 | Strain 6 | Strain 7 | Strain 8 | Strain 9 | Strain 10 |
| Ex. 16 | 0.39 | 0.78 | >25 | 0.39 | 0.78 | 0.20 | 0.39 | | | |
| Ex. 17 | 0.20 | 0.39 | >25 | 0.20 | 0.78 | 0.10 | 0.39 | | | |
| Ex. 18 | 0.39 | 0.39 | >25 | 0.20 | 0.39 | 0.20 | 0.20 | | | |
| Ex. 19 | 0.39 | 1.56 | >25 | 0.39 | 1.56 | 0.20 | 0.39 | | | |
| Ex. 20 | 0.20 | 0.39 | >25 | 0.20 | 0.20 | 0.20 | 0.78 | 0.10 | 0.39 | 0.39 |
| Ex. 21 | 0.20 | 0.20 | >25 | 0.39 | 0.39 | 0.20 | 0.78 | 0.20 | 0.20 | 0.39 |
| Ex. 22 | 0.39 | 0.39 | >25 | 0.39 | 0.39 | 0.39 | 3.13 | 0.20 | 0.20 | 0.39 |
| Ex. 23 | 0.78 | 0.78 | >25 | 0.39 | 0.39 | 0.20 | 3.13 | 0.39 | 0.20 | 0.39 |
| Ophroxacine | 0.78 | 0.78 | 0.10 | 0.78 | 12.5 | 25.0 | 0.78 | 0.78 | 0.78 | 1.56 |
| Amoxicillin | 0.05 | 0.025 | 6.25 | 0.10 | 0.10 | 0.025 | 0.05 | 0.025 | 0.025 | 0.05 |
| Strain means Objected strain | | | | | | | | | | |
| Strain 1: Helicobacter pylori ATCC 43504 | | | | | | | | | | |
| Strain 2: Helicobacter pylori ATCC 43625 | | | | | | | | | | |
| Strain 3: Escherichia coll NIH JC-2 | | | | | | | | | | |
| Strain 4: Helicobacter pylori No. 1 | | | | | | | | | | |
| Strain 5: Helicobacter pylori No. 2 | | | | | | | | | | |
| Strain 6: Helicobacter pylori No. 3 | | | | | | | | | | |
| Strain 7: Helicobacter pylori No. 4 | | | | | | | | | | |
| Strain 8: Hellcobacter pylori No. 19 | | | | | | | | | | |
| Strain 9: Helicobacter pylori No. 20 | | | | | | | | | | |
| Strain 10: Helicobacter pylori No. 28 | | | | | | | | | | |

**Table 5**

| Compound | M I C (µg/ml) | | | |
|---|---|---|---|---|
| | Strain 11 | Strain 12 | Strain 13 | Strain 14 |
| Example 1 | 3.13 | 6.25 | 25 | 3.13 |
| Example 5 | >25 | >25 | >25 | >25 |
| Example 8 | > 25 | > 25 | > 25 | > 25 |
| Example 11 | 25 | >25 | > 25 | > 25 |
| Example 18 | 25 | >25 | >25 | >25 |
| Example 22 | >25 | > 25 | > 25 | >25 |
| Ophroxacine | 0.39 | 0.10 | 0.10 | 12.5 |
| Amoxicillin | 0.10 | 0.05 | 0.05 | 0.20 |
| * Strain means Objected strain. | | | | |
| * Strain 11: Staphylococcus aureus 209PJC | | | | |
| Strain 12: Staphylococcus aureus ATCC 6538 | | | | |
| Strain 13: Bacillus subtilis ATCC 6633 | | | | |
| Strain 14: Staphylococcus aureus No. 2 (MRSA) | | | | |

## Claims

1. A compound of the general formula (VIII) 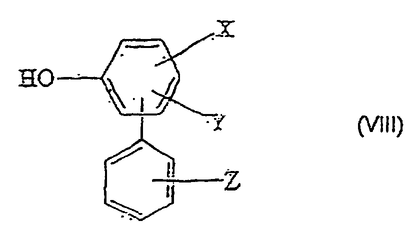 wherein X is hydrogen or halogen,
Y is hydrogen or substituted or unsubstituted aralkyloxycarbonyl group having 8 - 19 carbon atoms, wherein the aryl group may be substituted by one or more groups selected from alkyl groups having 1 to 10 carbon atoms, alkoxy having 1 to 10 carbon atoms, trihalogenomethyl or halogen,
Z is hydrogen, substituted or unsubstituted aralkyloxycarbonyl group having 8 - 19 carbon atoms, wherein the aryl group may be substituted by one or more groups selected from alkyl groups having 1 to 10 carbon atoms, alkoxy having 1 to 10 carbon atoms, trihalogenomethyl or halogen, or
Z is alkoxycarbonyl group having 2 - 19 carbon atoms, substituted in the alkyl group by a cycloalkyl group having 3 to 18 carbon atoms, wherein at least one of Y or Z is not hydrogen.

2. Compound according to daim 1, wherein Z is in the para position.

3. Compound according to claim 1, which is 4-(4-hydroxyphenyl)benzoate (4-methylbenzylester).

4. The compound 4-(4-hydroxyphenyl) benzoate [3-(4-fluorophenyloxy)propylester].

## Patentansprüche

1. Verbindung der allgemeinen Formel (VIII) 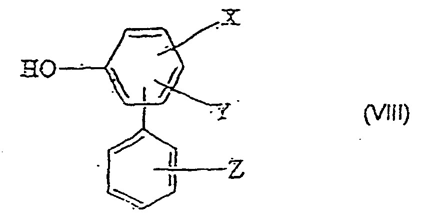 worin X ein Wasserstoff- oder Halogenatom ist,
Y ein Wasserstoffatom oder eine substituierte oder unsubstituierte Aralkyloxycarbonylgruppe mit 8 bis 19 Kohlenstoffatomen ist, wobei die Arylgruppe mit einer oder mehreren Gruppen substituiert sein kann, ausgewählt aus Alkylgruppen mit 1 bis 10 Kohlenstoffatomen, Alkoxygruppen mit 1 bis 10 Kohlenstoffatomen, Trihalogenmethylgruppe oder Halogenatom,
Z ein Wasserstoffatom oder eine substituierte oder unsubstituierte Aralkyloxycarbonylgruppe mit 8 bis 19 Kohlenstoffatomen ist, wobei die Arylgruppe mit einer oder mehreren Gruppen substituiert sein kann, ausgewählt aus Alkylgruppen mit 1 bis 10 Kohlenstoffatomen, Alkoxygruppen mit 1 bis 10 Kohlenstoffatomen, Trihalogenmethylgruppe oder Halogenatom, oder
Z eine Alkoxycarbonylgruppe mit 2 bis 19 Kohlenstoffatomen ist, die in der Alkylgruppe mit einer Cycloalkylgruppe mit 3 bis 18 Kohlenstoffatomen substituiert ist, wobei mindestens eine der Gruppen Y oder Z kein Wasserstoffatom ist.

2. Verbindung nach Anspruch 1, wobei Z in der para-Position vorliegt.

3. Verbindung nach Anspruch 1, die 4-(4-Hydroxyphenyl)benzoat(4-methylbenzylester) ist.

4. Die Verbindung 4-(4-Hydroxyphenyl)benzoat[3-(4-fluorphenyloxy)propylester].

## Revendications

1. Composé de formule générale (VIII) 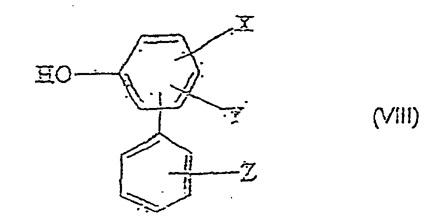 où X est l'hydrogène ou un halogène,
Y est l'hydrogène ou un groupe aralkyloxycarbonyle substitué ou non-substitué ayant 8 - 19 atomes de carbone, tandis que le groupe aryle peut être substitué par un ou plusieurs groupes choisis parmi les groupes alkyle ayant 1 à 10 atomes de carbone, alcoxy ayant 1 à 10 atomes de carbone, trihalogénométhyle ou halogène,
Z est l'hydrogène, un groupe aralkyloxycarbonyle substitué ou non-substitué ayant 8 - 19 atomes de carbone, tandis que le groupe aryle peut être substitué par un ou plusieurs groupes choisis parmi les groupes alkyle ayant 1 à 10 atomes de carbone, alcoxy ayant 1 à 10 atomes de carbone, trihalogénométhyle ou halogène, ou
Z est un groupe alcoxycarbonyle ayant 2 - 19 atomes de carbone, substitué dans le groupe alkyle par un groupe cycloalkyle ayant 3 à 18 atomes de carbone, tandis qu'au moins l'un des Y ou Z n'est pas l'hydrogène.

2. Composé selon la revendication 1, dans lequel Z est dans la position para.

3. Composé selon la revendication 1, qui est le 4-(4-hydroxyphényl)-benzoate ester de 4-méthylbenzyle.

4. Composé 4-(4-hydroxyphényl)benzoate ester de 3-(4-fluorophényloxy)propyle.
